# EUROPEAN PATENT SPECIFICATION

(11) **EP 3 957 368 B1**
(45) Date of publication and mention of the grant of the patent: **16.08.2023**
(21) Application number: 21180660.9
(22) Date of filing: 21.06.2021
(51) Int. Cl.: A61Q 19/00, A61K 8/891, A61K 8/892

(54) **FOOT OIL**
FUSSÖL
HUILE DE PIED

(30) Priority: 22.06.2020 EP 20181412
(43) Date of publication of application: 23.02.2022
(73) Proprietor: Regenboog-Den Hollander, Eveline Charlotte, 1731 RD Winkel (NL)
(72) Inventor: Regenboog-Den Hollander, Eveline Charlotte, 1731 RD Winkel (NL)
(74) Representative: van Breda, Jacobus

(56) References cited:
- EP-A1- 2 896 429
- Nn: "Technical Data Sheet XIAMETER(TM) PMX-1503 Fluid", , 1 January 2017 (2017-01-01), pages 1-3, XP055743480, Retrieved from the Internet: URL:https://www.ulprospector.com/documents /1163495.pdf?bs=2667&b=216587&st=20&r=eu&i nd=personalcare [retrieved on 2020-10-26]
- Nn: "SILSOFT * 8812 dimethicone and dimethiconol", , 1 March 2012 (2012-03-01), pages 1-4, XP055743498, Retrieved from the Internet: URL:https://www.ulprospector.com/documents /1156089.pdf?bs=2601&b=212150&st=20&r=eu&i nd=personalcare [retrieved on 2020-10-26]
- Retailer Nn: "Anti-Friction Gel Record ID 2019788", , 1 March 2013 (2013-03-01), pages 1-2, XP055743598, Retrieved from the Internet: URL:https://www.gnpd.com/sinatra/recordpag e/2019788/from_search/VmgJWPOPRN/?page=1 [retrieved on 2020-10-26]
- Nn: "No More Blisters Record ID 5431585", , 1 February 2018 (2018-02-01), pages 1-2, XP055743629, Retrieved from the Internet: URL:https://www.gnpd.com/sinatra/recordpag e/5431585/from_search/rcNRK1lBUy/?page=3 [retrieved on 2020-10-26]

## Description

The invention is directed to a foot oil.

The feet are under heavy load all day. Not only the joints and muscles in the feet, but also the skin of the feet needs extra attention and care. Blisters, inflammations, warm and irritated feet are often the cause of a stressed skin by wearing shoes. Further, foot and toe problems, like bunions (hallux valgus), hammer toes and mallet toes cause pain when walking, running or standing for a longer time.

New bought shoes are often tight and it is difficult to put on the shoes. The shoes do not fit well and this is caused by the fact that the feet cannot move properly in the shoes and will swell. Plasters and socks are not sufficient for a long term protection of the skin of the feet and protect the feet only temporarily and/or topical.

There are many foot gels and creams that keep the skin of the foot in good condition and protect it against dryness and cracking. However, these gels and creams do not protect the skin of the feet against friction.

The foot oil according to the present invention is surprisingly effective for the protection of the skin of the feet against friction. By using the foot oil according to the invention blisters, skin irritation and swelling of the foot will be strongly reduced or will not occur. Also, the pain caused by foot and toe problems will decrease.

The foot oil according to the invention consists of 90.0-99.9 wt% dimethicone, 10.0-0.1 wt% dimethiconol and optionally 1.10⁻⁶-0.1 wt% additives based on the total amount of foot oil.

Dimethicone and dimethiconol are silicone polymers and are known for use in cosmetic products, such as hair care products or skin care products.

Dimethicone is also known as polydimethylsiloxane (PDMS) or dimethylpolysiloxane. Dimethicone and dimethiconol are normally used in low concentrations up to 15 wt% in cosmetic products.

An example of a cosmetic foot product comprising polydimethylsiloxane and dimethiconol is described in CN106176264. The foot product described herein comprises only 2 wt% of polydimetylsiloxane and 3 wt% of dimethiconol.

The "Anti-Friction Gel" (Mintel Record ID 2019788) contains undisclosed amounts of cyclomethicone, dimethicone crosspolymer, cyclopentasiloxane, dimethiconol, resp., and parfum.

Dimethicone is a polymeric silicone with chemical structure A:

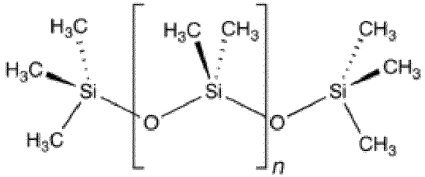

Dimethiconol is similar to dimethicone in its chemical structure, except that molecules of dimethiconol end with hydroxy (-OH) groups. This means that in the end groups of chemical structure A one of the -CH₃ groups is replaced by an -OH group.

In structure A the repeating unit can occur n times. This means that dimethicone and/or dimethiconol in the foot oil can be one silicone type with a fixed amount of repeating units. However, dimethicone and/or dimethiconol can also be present as a mixture of different silicone polymers with varying amounts of n repeating units. These silicone polymers will have different lengths and also different proper-ties.

In the foot oil according to the invention the amount of dimethicone is 90.0-99.9 wt%, preferably 91-99 wt% and more preferably 92-97 wt% based on the total amount of foot oil.

The amount of dimethiconol in the foot oil according to the invention is 10.0-0.1 wt%, preferably 9.0-1.0 wt% and more preferably 8.0-3.0 wt% based on the total amount of foot oil.

The optional amount of additives in the foot oil according to the invention is 1.10⁻⁶-0.1 wt% based on the total amount of foot oil. Additives can, for example, be fragrances, perfumes, aromatic substances, colorants, dyes, pigments, stabilizers, preservatives and antioxidants; like vitamin C and E.

The foot oil preferably has a density of 0.920 to 0.940 g/cm3, more preferably a density of 0.922 to 0.938 g/cm³.

The invention is also directed to the preparation of the foot oil. The foot oil is prepared by mixing at least one dimethicone, at least one dimethiconol and optionally the additives to form the foot oil. Preferably, at least one dimethicone has a density of 0.895 to 0.925 g/cm³, more preferably all dimethicones have a density of 0.895 to 0.925 g/cm³.

The invention if further directed to the foot oil for use in the protection of the skin against friction. Friction of the skin of the foot can be caused by footwear, for example boots, shoes, pumps, slippers and clogs. But also foot aids, for example a bandage, a plaster bandage, tape, a brace, a splint, an orthosis or a prosthesis can cause friction. The foot oil according to the invention can also be used in combination with these foot aids. The foot oil does not harm the footwear or the foot aids and will not cause stains. The foot oil is effective against friction when the foot is in direct contact with the footwear or the foot aid, but can also be used when wearing socks or a pantyhose.

By the use of the foot oil according to the invention the friction on the skin of the foot is reduced. This is also an advantage when foot and/or toe problems exist. Examples of foot and toe problems are callus, corns, blisters, bunions (hallux valgus), arthritis of the foot and/or ankle, plantar fasciitis, heel spur, bursitis in the heel, hammer toes, claw toes and mallet toes. The pain caused by these conditions will decrease when the foot oil according to the invention is used.

The foot oil according to the invention does not dissolve in water and also protects against friction in moist and/or wet conditions.

The foot oil according to the invention does not dry out and is also used in moisturizing the skin and in the prevention of callus.

Because the foot oil does not dry out the protection against friction remains present for at least 1 hour, preferably for at least 2 hours, more preferably for at least 4 hours. The protection against friction remains for at most 12 hours, preferably at most 18 hours, more preferably for at most 24 hours.

The foot oil according to the invention can be applied on the skin of the foot once a day, but also several times during a day. This depends on the activity of a person during the day. For example, when a person is walking, running or standing for several hours it is likely that the foot oil will be applied more than once. The foot oil can be removed by washing the feet with soap.

The property of the foot oil to protect against friction can not only be used on the feet, but also on other places of the body where clothes touch the skin and cause friction or where skin touches skin and causes friction during exercise or sport activities. The foot oil can, for example, also be used under the armpits, in the neck and between the legs during walking or running, and on the buttocks, for example during cycling.

The invention is further described by the accompanying examples, which are exemplary and explanatory of nature and are not limiting the scope of the invention. To the person skilled in the art it may be clear that many variants, being obvious or not, may be conceivable falling within the scope of protection, defined by the present claims.

### EXAMPLES

### Ingredients

### Dimethicone

D1: dimethicone with a density of 0.915 g/cm³
D2: dimethicone with a density of 0.903 g/cm³

### Mixtures

M1: dimethicone (87 wt%) and dimethiconol (13 wt%) with a density of 0.923 g/cm³
M2: cyclopentasiloxane (85%) and dimethiconol (15%) with a density of 0.963 g/cm³.

### Additive

A mixture of different fragrances and aromatic substances.

### Preparation

### Example 1

30 wt% of D1, 19.9 wt% of D2 and 50 wt% of M1 were mixed and 0.1 wt% additives were added. The mixture was blended and the density of the mixture was determined.

### Example 2

99.9 wt% of M1 and 0.1 wt% additives were blended and the density of the mixture was determined.

### Example 3

43 wt% of D1, 37 wt% of M1 and 20 wt% of M2 were mixed and the additives were added. The mixture was blended and the density of the mixture was determined.

### Example 4

52 wt% of D1, 37wt% of M1 and 4 wt% of M2 were mixed and the additives were added. The mixture was blended and the density of the mixture was determined.

**Table 1**

| **Example** | **Oil** | | | | **Density** |
|---|---|---|---|---|---|
| | Dimethicone (wt%) | Dimethico nol (wt%) | Cyclopentasi loxane (wt%) | Additives (wt%) | (g/cm3) |
| 1 | 93.40 | 6.50 | -- | 0.10 | 0.920 |
| 2 | 87.0 | 12.90 | -- | 0.10 | 0.923 |
| 3 | 75.09 | 7.81 | 17.00 | 0.10 | 0.930 |
| 4 | 90.18 | 6.32 | 3.40 | 0.10 | 0.925 |

### Results

The foot oils according to Examples 1-4 were applied on the skin of the feet and between the toes of several test persons. Shoes were put on and were worn for at least 10 hours. The amount of friction experienced during wearing was determined by the test persons wearing the shoes. An average of the friction that was determined by the test persons is given in Table 2.

The friction was determined as follows:
High friction = --
Medium friction = +/-
Low friction = ++

Also the time the low friction feel lasted was monitored by the test persons. The shortest time that was determined is given in Table 2.

The test persons also judged the feeling of the oil during application of the oil on the skin of the feet.

The feeling was determined as follows:
Wet, silky or greasy.

The test persons further judges the amount of callus on their feet after 30 days of daily use of the foot oil.

The amount of callus was determined as follows:
Improved; less callus than before the use of the foot oil.
Same; about the same amount of callus than before the use of the foot oil.

**Table 2**

| **Example** | **Properties** | | | |
|---|---|---|---|---|
| | Friction | Time (hours) | Feeling during application | Amount of callus |
| 1 | ++ | 10 | Silky | Improved |
| 2 | +/- | 2 | Silky | Same |
| 3 | +/- | 6 | Greasy | Same |
| 4 | +/- | 8 | Silky | Same |

## Claims

1. Foot oil consisting of 90.0-99.9 wt% dimethicone, 10.0-0.1 wt% dimethiconol and optionally 1.10⁻⁶-0.1 wt% additives based on the total amount of foot oil.

2. Foot oil according to claim 1, wherein the amount of dimethicone is 91-99 wt%, preferably 92-97 wt%.

3. Foot oil according to any one of the preceding claims, wherein the amount of dimethiconol is 9.0-1.0 wt%, preferably 8.0-3.0 wt%.

4. Foot oil according to any one of the preceding claims, wherein the foot oil has a density of 0.920 to 0.940 g/cm³, preferably 0.922 to 0.938 g/cm³.

5. Foot oil according to any one of the preceding claims, wherein the additives are aromatic substances, perfumes and/or combinations thereof.

6. Process for the preparation of a foot oil according to any one of claims 1-5, wherein at least one dimethicone, at least one dimethiconol and optionally the additives are mixed to form the foot oil.

7. Process according to claim 6, wherein at least one dimethicone has a density of 0.895 to 0.925 g/cm³.

8. Process according to claim 7, wherein all dimethicones have a density of 0.895 to 0.925 g/cm³.

9. Foot oil according to any one of claims 1-5, for use in the protection of the skin against friction.

10. Foot oil according to claim 9, wherein the protection against friction remains present for at least 1 hour.

11. Foot oil according to claim 9 or 10, wherein the friction is caused by footwear.

12. Foot oil according to any one of claims 1-5, for use in moisturizing the skin.

13. Foot oil according to any one of claims 1-5, for use in the prevention of callus.

## Patentansprüche

1. Fußöl bestehend aus 90,0-99,9 Gew.-% Dimethicon, 10,0-0,1 Gew.-% Dimethiconol und optional 1,10⁻⁶-0,1 Gew.-% Additive, bezogen auf die Gesamtmenge des Fußöls.

2. Fußöl gemäß Anspruch 1, wobei die Menge an Dimethicon 91-99 Gew.-%, bevorzugt 92-97 Gew.-% beträgt.

3. Fußöl gemäß einem der vorhergehenden Ansprüche, wobei die Menge an Dimethiconol 9,0-1,0 Gew.-%, bevorzugt 8,0-3,0 Gew.-% beträgt.

4. Fußöl gemäß einem der vorhergehenden Ansprüche, wobei das Fußöl eine Dichte von 0,920 bis 0,940 g/cm³, bevorzugt 0,922 bis 0,938 g/cm³ aufweist.

5. Fußöl gemäß einem der vorhergehenden Ansprüche, wobei die Additive Aromastoffe, Duftstoffe und/oder Kombinationen davon sind.

6. Verfahren zur Herstellung eines Fußöls gemäß einem der Ansprüche 1 bis 5, wobei mindestens ein Dimethicon, mindestens ein Dimethiconol und optional die Additive gemischt werden, um das Fußöl zu bilden.

7. Verfahren gemäß Anspruch 6, wobei mindestens ein Dimethicon eine Dichte von 0,895 bis 0,925 g/cm³ aufweist.

8. Verfahren gemäß Anspruch 7, wobei alle Dimethicone eine Dichte von 0,895 bis 0,925 g/cm³ aufweisen.

9. Fußöl gemäß einem der Ansprüche 1 bis 5 zur Verwendung zum Schutz der Haut gegen Reibung.

10. Fußöl gemäß Anspruch 9, wobei der Schutz gegen Reibung für mindestens 1 Stunde erhalten bleibt.

11. Fußöl gemäß Anspruch 9 oder 10, wobei die Reibung durch das Schuhwerk verursacht wird.

12. Fußöl gemäß einem der Ansprüche 1 bis 5 zur Verwendung zum Befeuchten der Haut.

13. Fußöl gemäß einem der Ansprüche 1-5 zur Verwendung zur Vorbeugung von Hornhaut.

## Revendications

1. Huile pour pieds constituée de 90,0 à 99,9 % en poids de diméthicone, de 10,0 à 0,1 % en poids de diméthiconol et facultativement de 1,10⁻⁶ à 0,1 % en poids d'additifs sur la base de la quantité totale d'huile pour pieds.

2. Huile pour pieds selon la revendication 1, dans laquelle la quantité de diméthicone est de 91 à 99 % en poids de préférence de 92 à 97 % en poids.

3. Huile pour pieds selon l'une quelconque des revendications précédentes, dans laquelle la quantité de diméthiconol est de 9,0 à 1,0 % en poids de préférence de 8,0 à 3,0 % en poids.

4. Huile pour pieds selon l'une quelconque des revendications précédentes, dans laquelle l'huile pour pieds a une densité de 0,920 à 0,940 g/cm³, de préférence de 0,922 à 0,938 g/cm³.

5. Huile pour pieds selon d'une quelconque des revendications précédentes, dans laquelle les additifs sont des substances aromatiques, des parfums et/ou des combinaisons de ceux-ci.

6. Procédé de préparation d'une huile pour pieds selon l'une quelconque des revendications 1 à 5, dans lequel au moins une diméthicone, au moins un diméthiconol et facultativement les additifs sont mélangés pour former l'huile pour pieds.

7. Procédé selon la revendication 6, dans lequel au moins une diméthicone a une densité de 0,895 à 0,925 g/cm³.

8. Procédé selon la revendication 7 dans lequel toutes les diméthicones ont une densité de 0,895 à 0,925 g/cm³.

9. Huile pour pieds selon l'une quelconque des revendications 1 à 5, pour son utilisation dans la protection de la peau contre le frottement.

10. Huile pour pieds selon la revendication 9, dans laquelle la protection contre le frottement reste présente pendant au moins 1 heure.

11. Huile pour pieds selon la revendication 9 ou 10, dans laquelle le frottement est provoqué par une chaussure.

12. Huile pour pieds selon l'une quelconque des revendications 1 à 5, pour son utilisation dans l'hydratation de la peau.

13. Huile pour pieds selon l'une quelconque des revendications 1 à 5, pour son utilisation dans la prévention d'un durillon.
